(19) Europäisches Patentamt
European Patent Office
Office européen des brevets

(11) Veröffentlichungsnummer: **0 226 947**
**A1**

(12) # EUROPÄISCHE PATENTANMELDUNG

(21) Anmeldenummer: 86117183.3

(22) Anmeldetag: 10.12.86

(51) Int. Cl.⁴: C 07 D 233/70
C 07 D 487/04, A 01 N 43/50
A 01 N 43/90

(30) Priorität: 21.12.85 DE 3545597

(43) Veröffentlichungstag der Anmeldung:
01.07.87 Patentblatt 87/27

(84) Benannte Vertragsstaaten:
AT BE CH DE ES FR GB GR IT LI NL SE

(71) Anmelder: CELAMERCK GmbH & Co. KG
Binger Strasse 173
D-6507 Ingelheim am Rhein(DE)

(72) Erfinder: Buck, Wolfgang, Dr.
In der Dörrwiese 37
D-6507 Ingelheim am Rhein(DE)

(72) Erfinder: Garrecht, Manfred, Dr.
Im Graben 4
D-6501 Wackernheim(DE)

(72) Erfinder: Schneider, Gerhard
Schleifmühlenweg 7a
D-6109 Mühltal 1(DE)

(72) Erfinder: Drandarevski, Christo, Dr.
Boehringer Strasse 8
D-6507 Ingelheim am Rhein(DE)

(54) **Neue herbizid wirksame Imidazolinone.**

(57) Imidazolinone der Formel

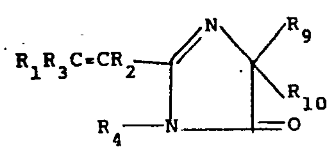

(Ia)

und (I)

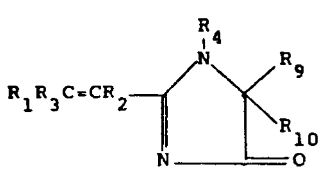

(Ib)

in denen $R_1$, $R_2$, $R_3$, $R_4$, $R_9$ und $R_{10}$ die im Text erläuterte Bedeutung haben können, nach üblichen Methoden hergestellt und als herbizide Wirkstoffe verwendet werden.

EP 0 226 947 A1

Die Erfindung betrifft Imidazolinone der Formel I

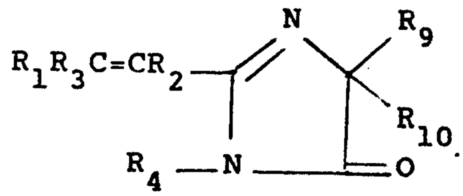

(Ia)

und

(I)

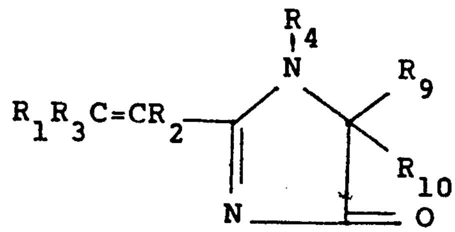

(Ib)

in denen

$R_1$ und $R_2$ unabhängig voneinander für gegebenenfalls substituiertes Niederalkyl,

$R_3$ für COOR$_5$, COR$_6$, CN oder COOM,

$R_4$ für H, gegebenenfalls substituiertes Niederalkyl, gegebenenfalls substituiertes Niederalkenyl oder $C_1$-$C_4$-Alkanoyl

$R_5$ für H, gegebenenfalls substituiertes $C_1$-$C_8$-Alkyl oder gegebenenfalls substituiertes Niederalkenyl, Phenyl oder $C_3$-$C_7$-Cycloalkyl,

$R_6$ für $NR_7R_8$, gegebenenfalls substituiertes Morpholinyl, gegebenenfalls substituiertes Pyrrolidinyl, gegebenenfalls substuiertes Piperidinyl, $NH-NH_2$, $NH-N=CR_{11}R_{12}$ oder

NH-N=C 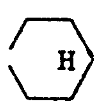

$R_7$ und $R_8$ unabhängig voneinander für H, gegebenenfalls

substituiertes Niederalkyl, Niederalkenyl oder gegebenenfalls substituiertes Phenyl, $R_7$ auch für OH,

$R_9$ und $R_{10}$ unabhängig voneinander für Niederalkyl

$R_{11}$ für H oder Niederalkyl,

$R_{12}$ für Niederalkyl,

M für ein Äquivalent eines Alkali-, Ammonium- oder Erdalkaliions, oder

$R_3$ und $R_4$ auch gemeinsam für -CO- stehen.

Hervorzuheben sind Imidazolinone der Formel

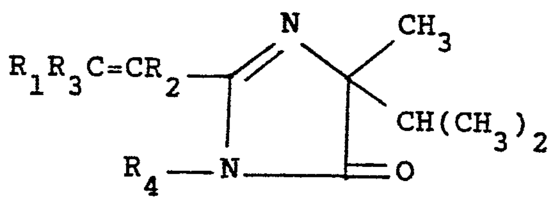

(Ia')

und Imidazoline der Formel

(I')

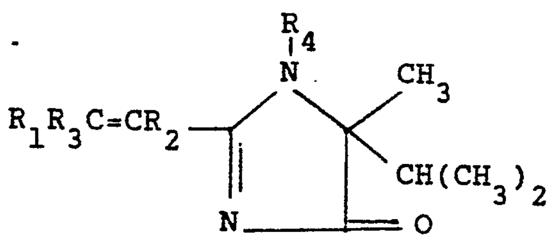

(Ib')

in denen

$R_1$ und $R_2$ unabhängig voneinander für Niederalkyl,

$R_3$ für $COOR_5$, $COR_6$, CN,

$R_4$ für H oder $C_1$-$C_4$-Alkanoyl,

$R_5$ für H, Niederalkyl oder Niederalkenyl oder $C_5$-$C_7$-Cycloalkyl,

$R_6$ für $NR_7R_8$, Morpholinyl, Pyrrolidinyl oder Piperidinyl,

| | |
|---|---|
| $R_7$ und $R_8$ | unabhängig voneinander für H, Niederalkyl, oder Phenyl, |
| | $R_7$ außerdem für OH, |
| $R_3$ und $R_4$ | gemeinsam auch für -CO- stehen. |

Im Rahmen der vorstehenden Definitionen steht Niederalkyl bevorzugt für Methyl, Ethyl, n-Propyl und i-Propyl, Alkanoyl für Acetyl und Propionyl, Niederalkenyl für Allyl.

Die Verbindungen der Formel Ia' und Ib' leiten sich von den tautomeren Stammverbindungen Ia, Ib mit $R_4$ = H ab, wobei aus den beiden tautomeren Grundstrukturen Ia und Ib formell auch die beiden nachstehend beschriebenen 2H-Pyrrolo[1,2-a]imidazole(Ia", $R_4$ und $R_3$ = -CO-) sowie 3H-Pyrrolo[1,2-a]imidazole (Ib", $R_4$ undR$_3$ = -CO-) herleiten:

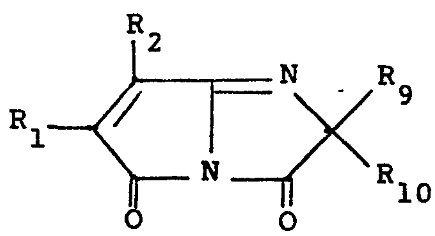

(Ia")

(I")

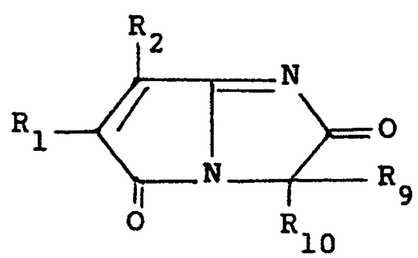

(Ib")

Im Text werden die Formeln Ia', Ia" bzw. Ib', Ib" sowie die die beiden Isomere umfassende Formel I je nach Bedeutung verwendet.

Niederalkylgruppen sind die $C_1$-$C_4$-Alkylradikale: Methyl, Ethyl, Propyl, Isopropyl, n-Butyl, sek.-Butyl, Isobutyl und tert.-Butyl.

Niederalkenylgruppen sind die $C_2$-$C_5$-Alkenylradikale wie etwa: Vinyl, Allyl

Die Niederalkyl-, Niederalkenyl oder Phenylgruppen gemäß vorstehender Definition können ein- oder mehrfach mit Halogen, Alkoxy- vorzugsweise Methoxy oder Ethoxy-, Hydroxy, Nitro, Cyano oder Mercapto substituiert sein.

Halogensubstituenten sind Fluor, Chlor, Brom und Jod, bevorzugt ist Fluor, Chlor und Brom. Von den halogensubstituierten Alkyl, Alkenyl und Phenylgruppen sind die Trifluormethyl, Trichlormethyl, Chloralkyl, o-, m- und p-Chlorphenyl sowie die 2,4-Dichlorphenylradikale zu nennen.

Substituierte Morpholinyl, Pyrrolidinyl- und Piperidinylgruppen sind beispielsweise die bis zu vierfach durch Niederalkyl, Halogen, Hydroxy oder Mercaptogruppen substituierten Heterocylen.

Alkaliionen sind die einwertigen Kationen von Lithium, Natrium, Kalium, Erdalkaliionen sind die zweiwertigen Kationen von Beryllium, Magnesium und Calcium, vorzugsweise Magnesium und Calcium.

Ammoniumionen sind die durch Protonierung oder Alkylierung von Aminen erhältlichen Kationen wie z.B. die Kationen von Triethanolamin, Methylamin, Dimethylamin, Trimethylamin, Ethylamin, Diethylamin, Triethylamin, Isopropylamin, Diisopropylamin.

Es sind bereits zahlreiche 2-Phenyl- bzw. 2-Hetaryl-substituierte 4-Isopropyl-4-methyl-4H-imidazol-1(3)H-5-one bekannt geworden. Diese Verbindungen sind herbizid wirksam. Sie befriedigen nicht immer im Hinblick auf Wirkungsstärke, Wirkungsprofil und Toxizität.

Demgegenüber zeigen die Verbindungen nach Formel I eine verbesserte herbizide Wirkung.

Die Verbindungen der Formel I können in an sich bekannter Weise hergestellt werden:

1. Durch Cyclisierung von Verbindungen der Formel

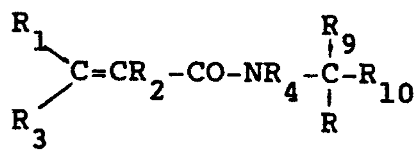

(II),

worin $R_1$, $R_2$, $R_3$, $R_4$, $R_9$ und $R_{10}$ die obige Bedeutung haben und R für $CONHR_4$ oder -CN steht, mit der Maßgabe, daß die beiden Reste $R_4$ unterschiedliche Bedeutung haben können.

Die Cyclisierung wird bewirkt durch wasserbindende Reagenzien, z.B. Dicyclohexylcarbodiimid und andere Kohlensäurediimide, Phosphoroxychlorid, Phosphorpentachlorid, Polyphosphorsäure, konzentrierte Schwefelsäure, Zinkchlorid, die auch bei den nachstehenden Ringschlußreaktionen Verwendung finden können, oder durch starke Basen in Gegenwart von $H_2O_2$. Vorzugsweise wird die Umsetzung bei Raumtemperatur durchgeführt.

2. Zur Herstellung von Verbindungen der Formel Ia, in denen $R_3$ und $R_4$ gemeinsam -CO- bedeuten (= Ia"), können Verbindungen der Formel

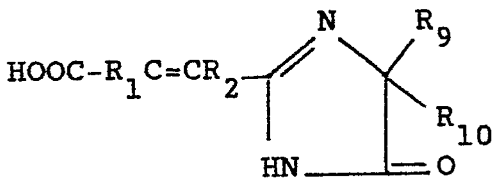
(III),

worin $R_1$, $R_2$, $R_9$ und $R_{10}$ die obige Bedeutung haben, in Gegenwart wasserbindender Mittel, wie etwa $PCl_5$, $POCl_3$, cyclisiert werden. Das wasserbindende Mittel, gegebenenfalls auch ein zusätzliches inertes Lösungsmittel, dient als Reaktionsmedium. Die Umsetzung erfolgt bevorzugt bei Raumtemperatur.

3. Zur Herstellung von Verbindungen der Formel Ib, in denen $R_3$ und $R_4$ gemeinsam -CO- bedeuten (= Ib"), können Verbindungen der Formel

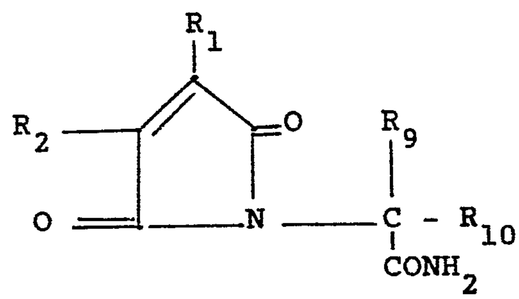
(IV)

worin $R_1$, $R_2$, $R_9$ und $R_{10}$ die obige Bedeutung haben, durch Einwirkung wasserbindender Mittel oder starker Basen, vorzugsweise bei Raumtemperatur, cyclisiert werden.

4. Zur Herstellung von Verbindungen der Formel I mit $R_4$ gleich H kann bei Verbindungen der Formel Ia"/Ib"

eine Ringöffnung durchgeführt werden. Mit Wasser oder wäßrigen Basen erhält man Verbindungen der Formel I, in denen $R_3$ COOH oder COOM bedeutet, mit Alkoholen $R_5OH$ erhält man Ester ($R_3$ gleich $COOR_5$), mit Aminen $R_6H$ erhält man Amide ($R_3$ gleich $COR_6$). Die Umsetzung erfolgt bei Temperaturen zwischen Raumtemperatur und der Siedetemperatur des Reaktionsgemischs, gegebenenfalls unter Zusatz eines Lösungsmittels, das die Reaktion nicht stört. Aus Verbindungen mit $R_3$ gleich $CONH_2$ können die entsprechenden Verbindungen mit $R_3$ gleich CN erhalten werden.

5. Zur Herstellung von Verbindungen der Formel I, in denen $R_4$ die obige Bedeutung hat, aber nicht für Wasserstoff steht, können Verbindungen der Formel

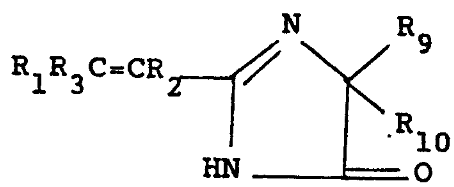

(Va)

bzw.

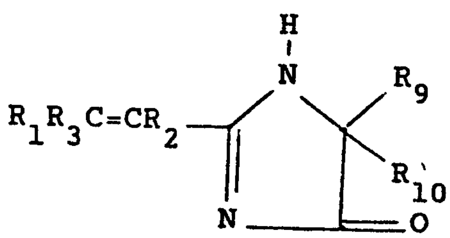

(Vb)

in der $R_1$, $R_2$, $R_3$, $R_9$ und $R_{10}$ die obige Bedeutung haben, mit einer Verbindung der Formel

$R_4X$ (VI),

in der $R_4$ die obige Bedeutung, ausgenommen Wasserstoff, hat und X für eine nucleophil

austauschbare Gruppe, wie Halogen, p-Toluolsulfonyl, Mesityl, steht oder $R_4X$ ein Carbonsäureanhydrid darstellt, alkyliert bzw. acyliert werden.
Die Umsetzung erfolgt im allgemeinen bei Temperaturen zwischen Raumtemperatur und der Siedetemperatur des Reaktionsgemisches, gegebenenfalls unter Zusatz eines Lösungsmittels.

6. Zur Herstellung von Verbindungen der Formel I, in denen $R_3$ $COOR_5'$ oder $COR_6$ bedeutet., worin $R_5'$ die Bedeutungen von $R_5$ außer H und $R_6$ die obigen Bedeutungen hat, werden entsprechende Verbindungen der Formel I mit $R_3$ gleich COOH, gegebenenfalls über reaktionsfähige Zwischenstufen, mit Alkoholen $R_5'OH$ bzw. Aminen $R_6H$ (bzw. Salzen dieser Verbindungen) verestert bzw. amidiert.

7. Verbindungen der Formel I, in denen $R_3$ für COOM steht, können aus den entsprechenden Carbonsäuren ($R_3$ = COOH) durch Umsetzung mit den Basen MOH hergestellt werden.

Die Ausgangsstoffe für die erfindungsgemäßen Verfahren können, soweit sie nicht bekannt sind, nach üblichen Methoden gewonnen werden, wie die nachstehenden Schemata zeigen; wenn nicht anders angegeben, haben die Substituenten die obigen Bedeutungen:

(1)

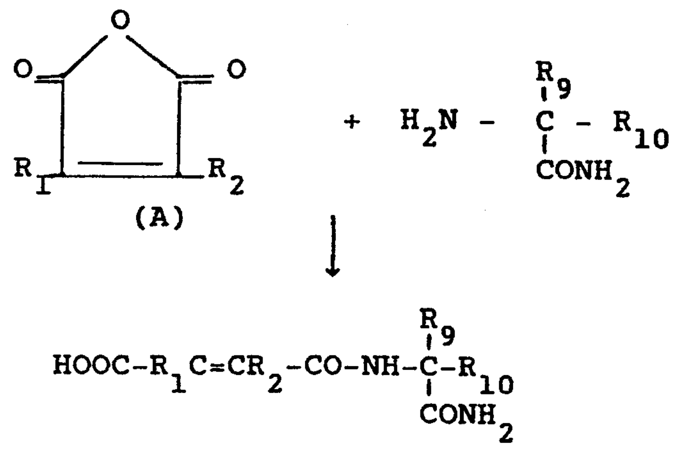

(A)

(B)

(2)

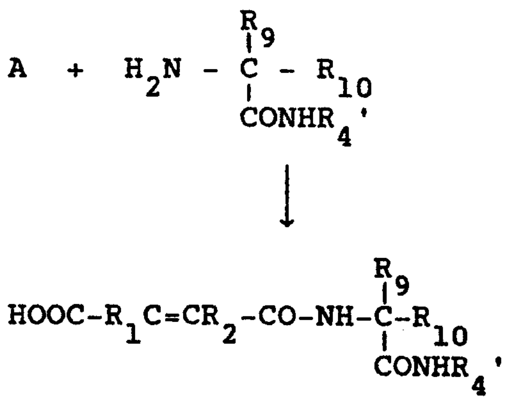

(C)

($R_4$' gleich gegebenenfalls substituiertes Niederalkyl bzw. Niederalkenyl)

(3)

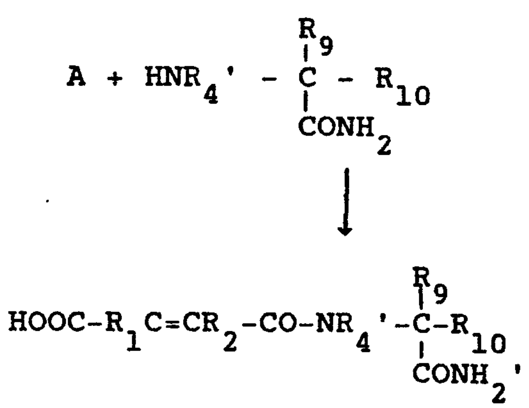

(D)

Die Reste $R_1$ und $R_2$ können jeweils auch die umgekehrten Positionen einnehmen. Dies gilt auch für die anderen hier beschriebenen Reaktionen, bei denen die Verknüpfung auf verschiedene Weise erfolgen kann. Zum Teil enthalten die erfindungsgemäßen

Verbindungen Asymmetriezentren, so daß sie als Racemate oder in Form von Enantiomeren vorliegen können.

Der Ringschluß bei den Verbindungen (B) führt zu III.

Ausgangsstoffe der Formel IV werden nach folgendem Schema erhalten:

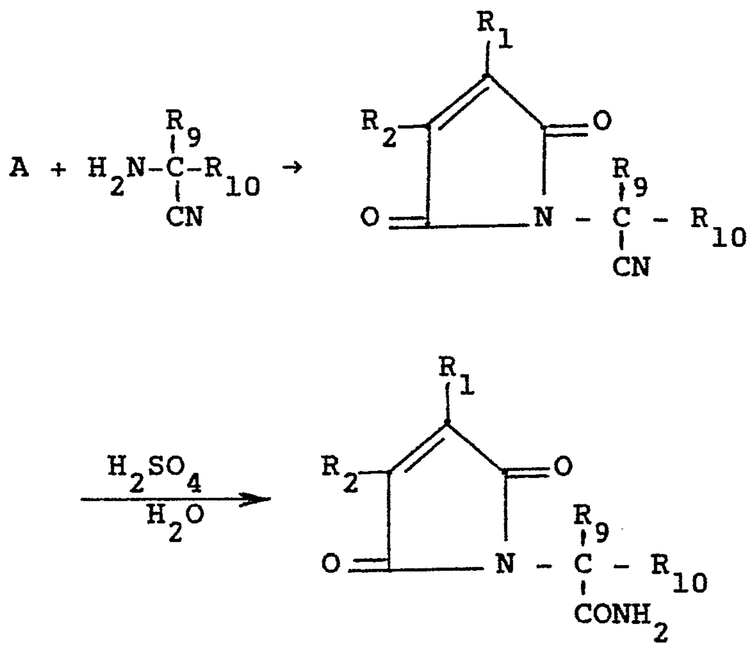


(IV)

Der Ringschluß bei C führt zu Verbindungen der Formel

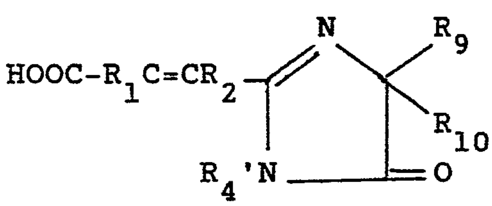

während der Ringschluß bei D Verbindungen der Formel

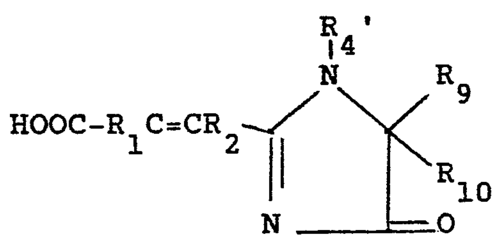

ergibt. In beiden Fällen handelt es sich um Endprodukte der Formel I, die ihrerseits Ausgangsstoffe für Endprodukte mit abgewandelter Carboxylgruppe darstellen.

Zur Herstellung der Ester der Formel I, worin $R_3$ die Gruppe $COOR_5'$ bedeutet und worin $R_1$, $R_2$, $R_5'$, $R_9$ und $R_{10}$ die obigen Bedeutungen haben, wird eine entsprechende Säure der Formel I, worin $R_3$ die Gruppe COOH bedeutet, in Gegenwart katalytischer Mengen einer starken Säure, z.B. p-Toluolsulfonsäure, in einem Überschuß des Alkohols $R_5'OH$ unter Erhitzen umgesetzt.

Des weiteren betrifft die Erfindung neue herbizide Mittel, welche mindestens einen Wirkstoff der Formel I enthalten, sowie Verfahren zur Unkrautbekämpfung.

Die Verbindungen der Formel I können in unveränderter Form oder vorzugsweise als Mittel mit den in der Formulierungstechnik üblichen Hilfsstoffen eingesetzt werden und zwar z.B. als Emulsionskonzentrate, Suspensionspulver, lösliche Pulver, Stäubemittel, Granulate oder direkt versprühbare Lösungen.

Die Wirkstoffe der Formel I enthaltenden Mittel werden nach an sich bekannten Verfahren der Formulierungstechnik z.B. durch Vermischen oder Vermahlen mit Streck- und gewünschtenfalls anderen Hilfmitteln hergestellt.

Als Streck- und Hilfsmittel sind zu nennen: Lösungsmittel, feste Trägerstoffe sowie gegebenenfalls oberflächenaktive Verbindungen.

Als Lösungsmittel können in Frage kommen: Aromatische

Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohle und Glykole sowie deren Aether und Ester, wie Methanol Aethanol, Propanol, Isopropanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder andere künstliche oder natürliche Polymere wie etwa Methylcellulose oder Ethylcellulose zugesetzt werden.

Als oberflächenaktive Verbindungen kommen je nach der Art des zu formulierenden Wirkstoffes der Formel I nichtionogene, kationen- und/oder anionenaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren

Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsprodukts.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage.

Als Beispiele nichtionischer Tenside seien Nonylphenolpolyäthoxyäthanole, Ricinusölpolyglykol-

äther, Polypropylen-Polyäthylenoxid-addukte, Tributylphenoxypolyäthoxyäthanol, Polyäthylenglykol und Octylphenoxypolyäthoxyäthanol erwähnt.

Die in der Formulierungstechnik gebräuchlichen Tenside sind u.a. in der folgenden Publikation beschrieben:

"Mc Cutcheon's Detergents and Emulsifiers Annual" MC Publishing Corp. Ridgewood, New Jersey, 1981;

Die herbiziden und die wachstumsregulatorischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides. Gewünschtenfalls werden die Zubereitungen zur Herstellung von Spritzbrühen mit Wasser verdünnt.

Insbesondere setzen sich bevorzugte Formulierungen folgendermassen zusammen: (% = Gewichtsprozent).

Emulgierbare Konzentrate:

| | |
|---|---|
| Verbindung der Formel I: | 1 bis 25 %, bevorzugt 5 bis 10 % |
| oberflächenaktive Mittel: | 5 bis 30 %, vorzugsweise 10 bis 20 % |
| flüssiges Trägermittel: | ad 100 % |

Stäube:

| | |
|---|---|
| Verbindung der Formel I: | 0,1 bis 10 %, vor zugsweise 0,1 bis 1 % |
| festes Trägermittel: | ad 100 % |

Suspensions-Konzentrate:

| | |
|---|---|
| Verbindung der Formel I | 5 bis 75 %, vorzugsweise 10 bis 50 % |
| oberflächenaktives Mittel: | 1 bis 40 %, vorzugsweise 2 bis 30 % |
| Wasser oder Wasser/Alkohol | ad 100 % |

Suspensionspulver:

| | |
|---|---|
| Verbindung der Formel I: | 0,5 bis 95 %, |
| oberflächenaktives Mittel: | 0,5 bis 20 %, vorzugsweise 1 bis 15 % |
| festes Trägermittel: | ad 100 % |

Granulate:

| | |
|---|---|
| Verbindung der Formel I: | 0,5 bis 30 %, vorzugsweise 3 bis 15 % |
| festes Trägermittel: | ad 100 % |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

FORMULIERUNGSBEISPIELE

(Angabe der Zusammensetzung in Gewichtsprozent)

1). Stäubemittel

0,3 % einer Verbindung der Formel I
1,0 % Methylcellulose
98,7 % Talkum

2) Suspensionspulver

25 % einer Verbindung der Formel I
55 % Kaolin
10 % Kolliodale Kieselsäure
9 % Calciumligninsulfonat
1 % Natriumtetrapropylenbenzolsulfonat

3) Suspensionspulver

95 % einer Verbindung der Formel I
4 % Calciumligninsulfonat
1 % Natriumtetrapropylenbenzolsulfonat

4) Emulsionskonzentrat

10 % einer Verbindung der Formel I
80 % Dimethylformamid
6,5 % Tensiofix AS (Emulgator)
3,5 % Tensiofix DS (Emulgator)

5) Suspensionskonzentrat

20 % einer Verbindung der Formel I
3 % Dispergiermittel z.B. Naphthalinsulfat-aldehyd-Copolymer-Na-Salz
1 % Bentone EW (Mondmorillomit)
0,2 % Entschäumer (Silicon)
0,05 % Konservierungsmittel
ad 100 Wasser

Aus den Konzentraten 2. und 4. werden durch Vermischen mit Wasser Spritzbrühen hergestellt, die im allgemeinen zwischen 0,05 und 0,5 % Wirkstoff enthalten.

Die erfindungsgemäßen Wirkstoffe, bzw. Mittel können als Defoliants, Desiccants, Krautabtötungsmittel, Unkrautvernichtungsmittel oder als wachstumsregulatiorische Mittel verwendet werden. Unter Unkräutern sind im weitesten Sinne alle Pflanzen zu verstehen, welche an Orten aufwachsen, wo sie unerwünscht sind.
Die Wirkung der erfindungsgemäßen Substanzen als totale oder selektive Herbizide oder als wachstumsregulatorische Mittel hängt im wesentlichen von der Aufwandmenge, Behandlungsart, Behandlungszeitpunkt sowie von der Formulierung ab.

Die erfindungsgemäßen Wirkstoffe können z.B. bei folgenden Pflanzen verwendet werden ohne daß durch nachstehender Auflistung eine Beschränkung der Anwendung auf die genannten Gattungen gegeben ist.

Dikotyle Unkräuter der Gattungen:
Sinapis. Lepidium, Galium, Stellaria, Matricaria, Anthemis, Galinsoga, Chenopodium, Urtica, Senecio,

Amaranthus, Portulaca, Xanthium, Convolvulus, Ipomoea, Polygonum, Sesbanis, Ambrosia, Cirsiu, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:
Gossypium, Glycine, Beta, Daucus, Phaseolus, Pisum, Solanum, Linum, Ipomoea, Vicia, Nicotiana, Lycopersicon, Arachis, Brassica, Lactura, Cucumis, Cucurbita.

Monokotyle Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischeamum, Sphenoclea, Dactyloctenium, Agrostis, Alopecurus, Apera.

Monokotyle Kulturen der Gattungen:
Oryza, Zea, Triticum, Hordeum, Avena, Secale, Sorghum, Panicum, Saccharum, Ananas, Asperagus, Allium.

Die erfindungsgemäßen Wirkstoffe können im Vor- und Nachauflauf in Nutzpflanzenkulturen eingesetzt werden, wie z.B. in Getreide, Baumwolle, Soja oder Kartoffeln oder in Dauerkulturen, wie z.B. in Forst- oder Ziergehölzen und im Obst-, Wein-, Hopfen- oder Beerenfruchtanbau.

BEISPIELE

1) 2-(2-Carboxy-1-methyl-prop-1-enyl)-4-isopropyl-4-methyl-4H-imidazol-1(3)H-5-on (I)

1a) 2,3-Dimethyl-N-(1-aminocarbonyl-1,2-dimethylpropyl)-maleinsäuremonoamid (II)

Dimethylmaleinsäureanhydrid (46 g) und 2-Amino-2,3-dimethylbuttersäureamid (48 g) werden in Methylenchlorid (70 ml) 15 min. zum Sieden erhitzt. Nach Abdestillieren eines Teils des Lösungsmittels wird mit Diethylether (300 ml) verrührt und das dabei ausfallende Produkt abgesaugt.
Man isoliert die Titelverbindung (90 g; 96 %) als kristallinen Feststoff vom Schmp. 70-72°C.

1b) 2-(2-Carboxy-1-methyl-prop-1-enyl)-4-isopropyl-4-methyl-4H-imidazol-1(3)H-5-on (I)

2,3-Dimethyl-N-(1-aminocarbonyl-1,2-dimethylpropyl)-maleinsäuremonoamid (II) (34,6 g) werden in Acetonitril (150 ml) suspendiert, mit Bicyclohexyl-carbonsäurediimid (30,9 g) versetzt und drei Tage bei Raumtemperatur gerührt. Es wird von dem ausgefallenen Harnstoff abgetrennt, das Filtrat wird eingeengt und an Kieselgel mit Aceton/Heptan (1:1) gereinigt.
Man isoliert die Titelverbindung (27,4 g; 86 %) als farblose Kristalle vom Schmp. 126-9°C.

Vorteilhaft wird die Reaktionsfolge 1a/1b ohne Isolierung der 1.Stufe als Eintopfreaktion durchgeführt.

Aus der so erhältlichen Carbonsäure der Formel I können die Salze wie folgt dargestellt werden.

2) 2-(2-Carboxy-1-methyl-prop-1-enyl)-4-isopropyl-4-methyl-4H-imidazol-1(3)H-5-on (I) Ammoniumsalz

2-(2-Carboxy-1-methyl-prop-1-enyl)-4-isopropyl-4-methyl4H-imidazol-1(3)H-4-on (I) wird in wäßrig-methanolischer Lösung mit einer äquimolaren Menge an Ammoniak zu einer Lösung des Salzes der Titelverbindung umgesetzt und ohne weitere Isolierung für die biologischen Prüfungen verwendet.

Analog zu Beispiel 2 werden in wäßrig- methanolischer Lösung durch Umsetzung mit der entsprechenden Base die folgenden Salze in Lösung hergestellt und ohne weitere Isolierung für die biologischen Prüfungen verwendet.

3) 2-(2-Carboxy-1-methyl-prop-1-enyl)-4-isopropyl -4-methyl-4H-imidazol-1(3)H-5-on-Isoopropylammoniumsalz (I)

4) 2-(2-Carboxy-1-methyl-prop-1-enyl)-4-isopropyl -4-methyl-4H-imidazol-1(3)H-5-on-Isopropyl Dimethylammoniumsalz (I)

5) 2-(2-Carboxy-1-methyl-prop-1-enyl)-4-isopropyl-4-methyl-4H-imidazol-1(3)H-5-on Diethylammoniumsalz (I)

6) 2-(2-Carboxy-1-methyl-prop-1-enyl)-4-isopropyl-4-methyl-4H-imidazol-1(3)H-5-on Triethylammonium salz (I)

7) 2-(2-Carboxy-1-methyl-prop-1-enyl)-4-isopropyl-4 methyl-4H-imidazol-1(3)H-5-on n-Hexylammoniumsalz (I)

8) 2-(2-Carboxy-1-methyl-prop-1-enyl)-4-isopropyl-4-methyl-4H-imidazol-1(3)H-5-on Natriumsalz (I)

9) 2-(2-Carboxy-1-methyl-prop-1-enyl)-4-isopropyl-4 methyl-4H-imidazol-1(3)H-5-on Calciumsalz (I)

10) 2-(2-Carboxy-1-methyl-prop-1-enyl)-4-isopropyl-4
methyl-4H-imidazol-1(3)H-5-on
[Tri-(2-hydroxyethyl]-ammoniumsalz (I)

11) 2-Isopropyl-2,6,7-trimethyl-2H-pyrrolo[1,2-a]-
imidazol-3,5-dion(Ia)

2-(2-Carboxy-1-methyl-prop-1-enyl)-4-isopropyl-4-methyl-4H-imidazol-1(3)H-5-on (2,38 g) werden in $POCl_3$ (5 ml) mit $PCl_5$ (2,09 g) gerührt. Es bildet sich eine dunkle Lösung, die nach längerem Stehen eingeengt wird. Der Rückstand wird mit Chlorform/Eiswasser verrührt, die organische Phase wird abgetrennt, eingeengt und chromatographisch gereinigt.
Man isoliert die Titelverbindung als Öl, das beim Reiben erstarrt;
Fp. 98-99°C (1,55 g; 70%).

<u>Elementaranalyse:</u>

| | | | |
|---|---|---|---|
| ber | C: 65,45 % | H: 7,27 % | N: 12,73 % |
| gef | C: 63,96 % | H: 7,64 % | N: 12,76 % |

12) 3-Isopropyl-3,6,7-trimethyl-3H-pyrrolo[1,2-a]-
imidazol-2,5-dion (Ib)

1(1-Aminocarbonyl-2,3-dimethyl-propyl)-3,4-dimethyl-pyrrol-2,5-dion (4,5 g) werden in $POCl_3$ (8 ml) suspendiert und portionsweise mit $PCl_5$ (4,5 g) versetzt. Es wird noch 2 Stunden bei Raumtemperatur nachgerührt, eingeengt, in Chloroform/Eiswasser aufgenommen, die organische Phase abgetrennt und an Kieselgel mit Aceton/Heptan (1 : 1) gereinigt.

Man isoliert die Titelverbindung als gelbes Öl (3,3 g; 79 %)

Elementaranalyse:

ber. C: 65,45 % H: 7,27 % N: 12,73 %

gef. C: 63,93 % H: 7,24 % N: 12,26 %

13) 2-[2-(Morpholin-4-yl-carbonyl)-1-methyl-prop-1-enyl]-4-methyl-4-isopropyl-4H-imidazol-1(3)H-5-on (I)

Morpholin (5 ml) und 3-Isopropyl-3,6,7-trimethyl-3H-pyrrolo[1,2-a]imidazol-2,5-dion (Ib") (2,2 g) werden 30 Stunden in Acetonitril (10 ml) zum Sieden erhitzt. Nach dem Einengen des Reaktionsgemisches wird an Kieselgel mit Aceton/Heptan (1 : 1) gereinigt.

Man isoliert die Titelverbindung als hellbraunes öl (1,7 g)

Elementaranalyse:

ber. C: 62,60 % H: 8,15 % N: 13,70 %

gef. C: 62,28 % H: 8,31 % N: 13,28 %

14) 2-(2-Carboxy-1-ethyl-prop-1-enyl)-4-isopropyl-4-methyl-4H-imidazol-1(3)H-5-on (I)
und
2-(2-Carboxy-1-methyl-but-1-enyl)-4-isopropyl-4-methyl-4H-imidazol-1(3)H-5-on (I)

Zu einer Lösung von Ethyl-methyl-maleinsäure- anhydrid (7,0 g) und 2-Amino-2,3- dimethyl- buttersäurenitril (6,7 g) in Tetrahydrofuran wird innerhalb von 2 Stunden bei -15°C eine Lösung von Butyllithium in Hexan (31 ml einer 1,6 molaren Lösung) getropft, 30 Min. bei 0°C nachgerührt und dann mit Natronlauge (13 g; 50 % wäßrige Lösung) und danach mit Wasser

(30 ml) versetzt. Es wird von der organischen Phase abgetrennt und innerhalb von 45 Min. bei -10°C Perhydrol (24 g; 30 %ig) zugetropft. Es wird 3 Stunden bei 80°C gerührt, mit Schwefelsäure neutralisiert und mit Dichlormethan ausgeschüttelt. Aus der organischen Phase isoliert man ein gelbes Öl, welches beim Verrühren mit Ether auskristallisiert.

Man isoliert eine Mischung der Titelverbindungen (0,9 g) als weiße Kristalle vom Schmp. 155-7°C. Die Verbindung kann auch entsprechend Beispiel 1a/1b hergestellt werden.

15) 2-(2-Ethoxycarbonyl-1-methyl-prop-1-enyl)-4-isopropyl-4-methyl-4H-imidazol-1(3)H-5-on (I)

15a) 2,3-Dimethyl-N-(1-aminocarbonyl-1,2-dimethylpropyl)-maleinsäuremonoamid-ethylester (II)

Man isoliert die Titelverbindung (5,5 g; 97 %) nach mehrstündigem Erhitzen von 2-(2-Carboxy-1-methyl-prop-1-enyl)-4-isopropyl-4-methyl-4H-imidazol-1(3)H-5-on (I) (4,76 g) in Ethanol (50 ml) in Gegenwart katalytischer Mengen von p-Toluolsulfonsäure als farblose Kristalle vom Schmp. 113-116°C.

15b) 2-(2-Ethoxycarbonyl-1-methyl-prop-1-enyl)-4-isopropyl-4-methyl-4H-imidazol-1(3)H-5-on (I)

2,3-Dimethyl-N-(1-aminocarbonyl-1,2-dimethylpropyl)-maleinsäuremonoamid-ethylester (II) (4,3 g) werden in $POCl_3$ (6 ml) suspendiert, mit $PCl_5$ (3,1 g) versetzt und eine Stunde bei

Raumtemperatur gerührt, eingeengt, auf Eis gegossen, mit Dichlormethan versetzt, mit Natriumbicarbonat neutralisiert, die organische Phase isoliert, getrocknet und eingeengt. Der Rückstand wird an Kieselgel mit Aceton/Heptan (1 : 1) gereinigt.

Man isoliert die Titelverbindung (1,3 g; 33 %) als farblose Kristalle vom Schmp. 98-100°C.

16) 2-(2-Carbamyl-1-methyl-prop-1-enyl)-4-isopropyl-4-methyl-4H-imidazol-1(3)H-5-on (I)

2-Isopropyl-2,6,7-trimethyl-2H-pyrrolo[1,2-a]-imidazol-3,5-dion (2,66 g) werden im Autoklaven mit Ammoniak in Tetrahydrofuran (etwa 20 ml flüssiger Ammoniak in 30 ml Tetrahydro-furan) 10 Stunden auf 80°C erwärmt. Danach wird das Reaktionsgemisch filtriert, eingeengt und mit Diethylether verrieben.

Man isoliert die Titelverbindung (2,4 g) als bräunliche Kristalle vom Schmp. 187-191°C.

17) 2-(1-Methyl-2-morpholinocarbonylprop-1-enyl-4-isopropyl-4-methyl-4H-imidazol-1(3)H-5-on

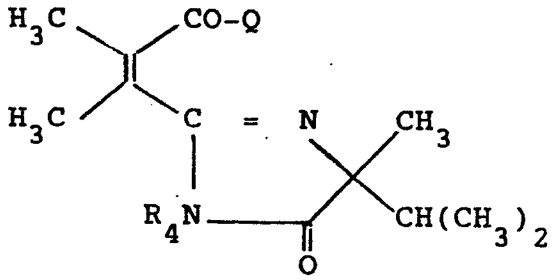

(Z)

Q: 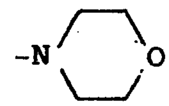

$R_4$: H

2,4 g 2-Isopropyl-2,6,7-trimethyl-2H-pyrrolo[1,2-a]-imidazol-3,5-dion werden zu einer Lösung von 14,3 g Morpholin in 150 ml abs. Methanol gegeben und 2 Stunden zum Rückfluß erhitzt. Man engt dann auf ca. 60 g ein, verdünnt mit Ether, saugt ab, wäscht mit Ether und trocknet.
Man erhält 26,6 g (80 % d.Th.) weißes, kristallines Produkt, Fp. 174-175°C.

18) 2-(2-Hydrazinocarbonyl-1-methylprop-1-enyl)-4-isopropyl-4-methyl-4H-imidazol-1(3)H-5-on

(Formel Z; Q: $NH-NH_2$, $R_4$: H)

5,4 g 2-Isopropyl-2,6,7-trimethyl-2H-pyrrolo-[1,2-a]imidazol-3,5-dion werden zu einer Lösung von 1,23 g Hydrazinhydrat in 50 ml Methanol gegeben. Man rührt 1/4 Stunde bei Raumtemperatur und dampft dann ein. Der Rückstand wird in Ether gelöst, kristallisiert, abgesaugt, gewaschen und getrocknet.
Man erhält 5,8 g (94 % d.Th.) einer weißen kristallinen Substanz, Fp. 163-165°C.

19) 2-(1-Methyl-2-n-propoxycarbonylprop-1-enyl)-1-acetyl-4-isopropyl-4-methyl-4H-imidazol-1H-5-on

(Formel Z; Q: $-O-n-C_3H_7$, $R_4$: $CH_3CO$)

2,8 g 1-(1-Methyl-2-n-propoxycarbonylprop-1- enyl)-4-isopropyl-4-methyl-4H-imidazol-1H-on werden in 30 ml Acetanhydrid 5 Stunden unter Rückfluß gerührt. Dann wird eingedampft und mit 800 ml Aceton/Heptan 1 : 1 über Kieselgel chromatographiert. Man erhält 2,8 g (87 % d.Th.) eines gelben öls.

<u>Elementaranalyse:</u>

| | | | |
|---|---|---|---|
| ber | C: 63,30 % | H: 8,07 % | N: 8,69 % |
| gef | C: 61,95 % | H: 8,24 % | N: 8,77 % |

Der entsprechende Ethylester (Formel Z; Q: $-OC_2H_5$; $R_4$: $CH_3CO$) wird entsprechend erhalten.
öl, Ausbeute 88 % d.Th.).

20) 2-(2-Ethylenhydrazocarbonyl-1-methylprop-1-enyl)-4-isopropyl-4-methyl-4H-imidazol-1-(3)H-5-on

(Formel Z; Q: $-NH-N=CH-CH_3$, $R_4$: H)

Eine Mischung aus 2,2 g des nach Beispiel 18 erhaltenen Produkts in 30 ml Methanol wird mit 2,2 g Acethaldehyd vermischt. Man läßt die Mischung 1/2 Stunde bei Raumtemperatur stehen, dampft ein, kristallisiert mit 30 ml Ether, saugt ab und trocknet. Man erhält 2,1 g (87 % d.Th.) weiße Kristalle, Fp. 173-174°C.

Analog erhält man die folgenden Verbindungen der Formel Z: $(R_4=H)$

| Q | Fp. [°C] |
|---|---|
| $-NH-N=C(CH_3)_2$ | 158 - 161 |
| 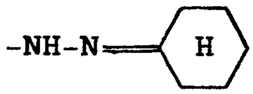 | 163 - 166 |

21) 2-(2-Ethoxycarbonyl-1-methylprop-1-enyl)-4-isopropyl-4-methyl-4H-imidazol-1(3)H-5-on

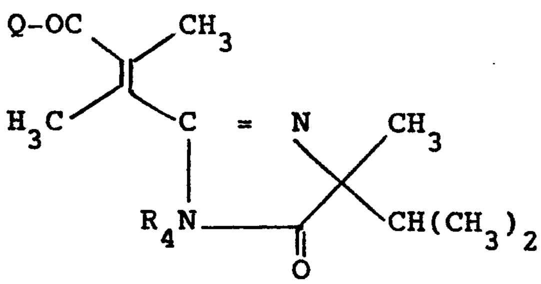

(Z')

Q: $OC_2H_5$

$R_4$: H

Zu einer Lösung von 12 g 2-Amino-2,3-dimethylbuttersäureamid und 12 ml Triethylamin in 80 ml Methylenchlorid tropft man unter Rühren und Eiskühlung eine Lösung von 13 g 2,3-Dimethylfumarsäuremonoethylesterchlorid in 20 ml Methylenchlorid. Man rührt 2 Stunden bei Raumtemperatur, schüttelt die Lösung mit Wasser, 2 N Salzsäure und wieder mit Wasser, trocknet sie und engt sie ein. Der Rückstand wird mit Diethylether verrieben, abgesaugt und aus wenig Ethanol umkristallisiert. Man erhält 8,0 g (41 % d.Th.) 2,3-Dimethylfumarsäuremonoethylester-N-(1-aminocarbonyl-1-methylisobutyl)- amid, Fp. 122-125°C.

8,0 g dieser Verbindung werden in ein Gemisch von 10 g $PCl_5$ und 50 ml $POCl_3$ eingetragen und 6 Stunden bei Raumtemperatur gerührt. Das Gemisch wird bei 60°C im Vakuum eingeengt, der Rückstand in Methylenchlorid aufgenommen, mit Wasser verrührt und mit Natriumhydrogencarbonat neutralisiert. Die Methylenchloridlösung wird abgetrennt, getrocknet und eingeengt. Man erhält 7,0 g (93 % d.Th.) eines hellgelben Öls, dessen Struktur spektroskopisch bestätigt wird.

22) 2-(1-Methyl-2-morpholinocarbonylprop-1-enyl)-4-isopropyl-4-methyl-4H-imidazol-1(3)H-5-on

(Formel Z'; Q: 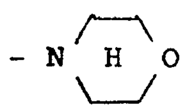 , $R_4$: H)

3,7 g des Endprodukts aus Beispiel 21 werden mit 4 ml Morpholin 2 Stunden bei 120°C gerührt. Das überschüssige Morpholin wird im Vakuum abdestilliert, der Rückstand chromatographisch gereinigt (80 g Kieselgel, Diisopropylether/Methanol 10 : 3). Man erhält 1,7 g öligen Rückstand, aus dem beim Verreiben mit Ether das Reaktionsprodukt, Fp. 145°C, erhalten wird.

In den nachstehenden Tabellen sind weitere erfindungsgemäß erhältliche Verbindungen aufgeführt:

## TABELLE I

Verbindungen der Formel

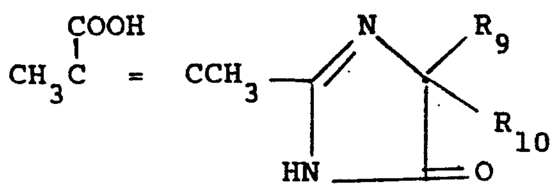

| Nr. | $R_9$ | $R_{10}$ | Fp. [°C] |
|---|---|---|---|
| 1 | $CH_3$ | $CH_3$ | 150 - 158 |
| 2 | $CH_3$ | $C_2H_5$ | 113 - 115 |
| 3 | $C_2H_5$ | $C_2H_5$ | 135 - 140 |
| 4 | $CH_3$ | $n-C_3H_7$ | 116 - 118 |

TABELLE II

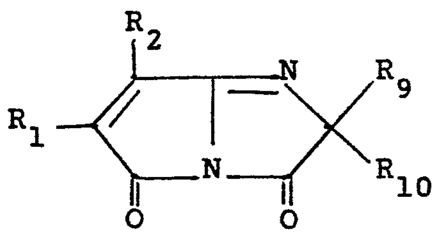

| Nr. | $R_1$ | $R_2$ | $R_9$ | $R_{10}$ | Fp. [°C] |
|---|---|---|---|---|---|
| 1 | $CH_3$ | $CH_3$ | $CH_3$ | $CH_3$ | 100-103 |
| 2 | $CH_3$ | $CH_3$ | $CH_3$ | $C_2H_5$ | 88-89 |
| 3 | $CH_3$ | $CH_3$ | $CH_3$ | $n-C_3H_7$ | 68-70 |
| 4 | $CH_3$ | $CH_3$ | $C_2H_5$ | $C_2H_5$ | 75-78 |
| 5* | $CH_3$ | $C_2H_5$ | $CH_3$ | $i-C_3H_7$ | Öl |

* Die Substituenten $R_1$ und $R_2$ können auch umgekehrt angeordnet sein, d.h. $R_1 = C_2H_5$, $R_2 = CH_3$.

TABELLE III

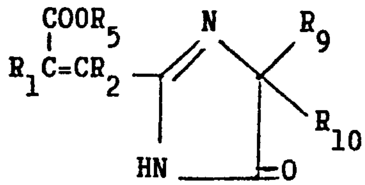

| Nr. | $R_1$ | $R_2$ | $R_5$ | $R_9$ | $R_{10}$ | Fp[C°] |
|---|---|---|---|---|---|---|
| 1 | $CH_3$ | $CH_3$ | $C_2H_5$ | $CH_3$ | $CH_3$ | |
| 2 | $CH_3$ | $CH_3$ | $C_2H_5$ | $CH_3$ | $C_2H_5$ | 95 |
| 3 | $CH_3$ | $CH_3$ | $CH_3$ | $CH_3$ | $i-C_3H_7$ | 128-131 |
| 4 | $CH_3$ | $CH_3$ | $C_2H_5$ | $CH_3$ | $i-C_3H_7$ | 100-101 |
| 5 | $CH_3$ | $CH_3$ | $n-C_3H_7$ | $CH_3$ | $i-C_3H_7$ | 84- 85 |
| 6 | $CH_3$ | $CH_3$ | $i-C_3H_7$ | $CH_3$ | $i-C_3H_7$ | 85- 87 |
| 7 | $CH_3$ | $CH_3$ | $n-C_4H_9$ | $CH_3$ | $i-C_3H_7$ | 82- 83 |
| 8 | $CH_3$ | $CH_3$ | $CH_2-CH_2-CH(CH_3)_2$ | $CH_3$ | $i-C_3H_7$ | 80- 83 |
| 9 | $CH_3$ | $CH_3$ | $n-C_8H_{17}$ | $CH_3$ | $i-C_3H_7$ | |
| 10 | $CH_3$ | $CH_3$ | 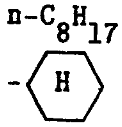 | $CH_3$ | $i-C_3H_7$ | 113-117 |
| 11 | $CH_3$ | $CH_3$ | $C_2H_5$ | $CH_3$ | $n-C_3H_7$ | 88-90 |
| 12 | $CH_3$ | $CH_3$ | $C_2H_5$ | $C_2H_5$ | $C_2H_5$ | |
| 13* | $CH_3$ | $C_2H_5$ | $CH_3$ | $CH_3$ | $i-C_3H_7$ | Öl |
| 14* | $CH_3$ | $C_2H_5$ | $C_2H_5$ | $CH_3$ | $i-C_3H_7$ | Öl |
| 15* | $CH_3$ | $C_2H_5$ | $n-C_3H_7$ | $CH_3$ | $i-C_3H_7$ | Öl |

*Es liegen Isomere vor, d.h. es kann auch $R_1$ $C_2H_5$ und $R_2$ $CH_3$ sein.

TABELLE IV

Verbindungen der Formel

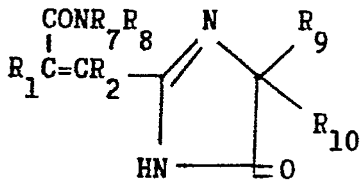

| Nr. | $R_1$ | $R_2$ | $R_7$ | $R_8$ | $R_9$ | $R_{10}$ | Fp[C°] |
|---|---|---|---|---|---|---|---|
| 1 | $CH_3$ | $CH_3$ | H | OH | $CH_3$ | i-$C_3H_7$ | 174-175 |
| 2 | $CH_3$ | $CH_3$ | H | $C_2H_5$ | $CH_3$ | i-$C_3H_7$ | 197-200 |
| 3 | $CH_3$ | $CH_3$ | H | i-$C_3H_7$ | $CH_3$ | i-$C_3H_7$ | 218-221 |
| 4 | $CH_3$ | $CH_3$ | H | n-$C_6H_{13}$ | $CH_3$ | i-$C_3H_7$ | 184-186 |
| 5 | $CH_3$ | $CH_3$ | H | 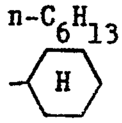 | $CH_3$ | i-$C_3H_7$ | 225-230 |
| 6 | $CH_3$ | $CH_3$ | H | $C_6H_5$ | $CH_3$ | i-$C_3H_7$ | 240-243 |
| 7 | $CH_3$ | $CH_3$ | $CH_3$ | $CH_3$ | $CH_3$ | i-$C_3H_7$ | 160-162 |
| 8 | $CH_3$ | $CH_3$ | $C_2H_5$ | $C_2H_5$ | $CH_3$ | i-$C_3H_7$ | 145-146 |
| 9 | $CH_3$ | $CH_3$ | $CH_2-CH=CH_2$ | $CH_2-CH=CH_2$ | $CH_3$ | i-$C_3H_7$ | 142-143 |
| 10 | $CH_3$ | $CH_3$ | $-(CH_2)_4-$ | | $CH_3$ | i-$C_3H_7$ | 159-161 |
| 11 | $CH_3$ | $CH_3$ | $-CH_2-CH_2-O-CH_2-CH_2-$ | | $CH_3$ | $CH_3$ | 158-160 |
| 12 | $CH_3$ | $CH_3$ | H | $CH(CH_3)_2$ | $CH_3$ | $C_2H_5$ | 170-175 |
| 13 | $CH_3$ | $CH_3$ | $-CH_2-CH_2-O-CH_2-CH_2-$ | | $CH_3$ | $C_2H_5$ | 158-160 |
| 14 | $CH_3$ | $CH_3$ | $-CH_2-CH_2-O-CH_2-CH_2-$ | | $C_2H_5$ | $C_2H_5$ | 167-170 |
| 15* | $CH_3$ | $C_2H_5$ | H | $CH_3$ | $CH_3$ | i-$C_3H_7$ | 161-165 |
| 16* | $CH_3$ | $C_2H_5$ | $-CH_2-CH_2-O-CH_2-CH_2-$ | | $CH_3$ | i-$C_3H_7$ | 127-130 |

*Es liegen Isomere vor, d.h. es kann auch $R_1$ $C_2H_5$ und $R_2$ $CH_3$ sein.

## T A B E L L E   A

Ausgangsstoffe der Formel

$$R'_3O-CO-\underset{CH_3}{C}=\underset{CH_3}{C}-CONH-\overset{CH_3}{\underset{CONH_2}{C}}-CH(CH_3)_2$$

| Nr. | $R_3'$ | Fp. [°C] |
|---|---|---|
| 1 | $C_2H_5$ | 117-118 |
| 2 | $n-C_3H_7$ | 125-126 |
| 3 | $i-C_3H_7$ | 124-126 |
| 4 | $n-C_4H_9$ | 115-116 |
| 5 | $CH_2CH_2CH(CH_3)_2$ | 79-80 |
| 6 | $n-C_8H_{17}$ | 84-85 |
| 7 | $CH_2CH=CH_2$ | |
| 8 | $CH_2CH_2-O-C_2H_5$ | 52-60 |
| 9 | 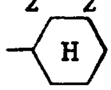 | 129-130 |

## TABELLE B

Ausgangsstoffe der Formel

$$R'_3\text{-CO-}R_1C{=}CR_2\text{-CONH-}\underset{\substack{|\\ CONH_2}}{\overset{\substack{R_9\\|}}{C}}\text{-}R_{10}$$

| Nr. | $R_1$ | $R_2$ | $R_9$ | $R_{10}$ | $R_3'$ | Fp.[°C] |
|---|---|---|---|---|---|---|
| 1 | $CH_3$ | $CH_3$ | $CH_3$ | $CH_3$ | $C_2H_5$ | |
| 2 | $CH_3$ | $CH_3$ | $CH_3$ | $C_2H_5$ | $C_2H_5$ | 142-145 |
| 3 | $CH_3$ | $CH_3$ | $CH_3$ | n-$C_3H_7$ | $C_2H_5$ | 138 |
| 4 | $CH_3$ | $CH_3$ | $C_2H_5$ | $C_2H_5$ | $C_2H_5$ | 150 |
| 5 | $CH_3$ oder $C_2H_5$ | $C_2H_5$ oder $CH_3$ | $CH_3$ | i-$C_3H_7$ | $CH_3$ | 65-67 |
| 6 | $CH_3$ oder $C_2H_5$ | $C_2H_5$ oder $CH_3$ | $CH_3$ | i-$C_3H_7$ | $CH_3$ | 68-70 |
| 7 | $CH_3$ oder $C_2H_5$ | $C_2H_5$ oder $CH_3$ | $CH_3$ | i-$C_3H_7$ | $CH_3$ | 75 |

Prüfung auf herbizide Wirkung

Die Schädigung der Unkautpflanzen bzw. die Kulturpflanzenverträglichkeit wurde in einem Schlüssel von 1 - 9 bonitiert.

Dabei bedeutet

| | |
|---|---|
| 1 | 100 % |
| 2 | 100 - 97,5 % |
| 3 | 97,5 - 95 % |
| 4 | 95 - 90 % |
| 5 | 90 - 85 % |
| 6 | 85 - 75 % |
| 7 | 75 - 65 % |
| 8 | 65 - 32,5 % |
| 9 | 32,5 - 0 % Wirkung |

1. Wirkung gegen Unkräuter

Samen bzw. Rhizomstücke mono- und dikotyler Unkräuter wurden in Einheitserde in Plastiktöpfen (Ø 9 cm) ausgelegt und mit Sand abgedeckt. Die als benetzbare Pulver bzw. als Emulsionskonzentrate formulierten erfindungsgemäßen Verbindungen wurden in Form wäßriger Suspensionen bzw. Emulsionen auf die Erdoberfläche appliziert. Die Wasseraufwandmenge pro Topf entsprach dabei umgerechnet 800 l/ha. Nach der Behandlung wurden die Versuchstöpfe im Gewächshaus aufgestellt und die Versuchspflanzen unter guten Wachstumsbedingungen (Temperatur: tags 20°C, nachts 15°C, rel. Luftfeuchte 60 - 80 %) kultiviert. Nach ca. 3 Wochen wurde die Pflanzenschädigung visuell bonitiert. Als Vergleich dienten dabei unbehandelte Kontrollen.

Die erfindungsgemäßen Verbindungen wiesen eine zum Teil ausgezeichnete herbizide Wirksamkeit gegen wirtschaftlich bedeutende mono- und dikotyle Unkräuter auf.

In ähnlicher Weise werden verschiedene Unkräuter in Töpfen im Gewächshaus bis zum 2 - 6 Blattstadium herangezogen und dann im Nachauflaufverfahren mit den erfindungsgemäßen Verbindungen behandelt. Nach ca. 3 - 4 Wochen wurden die Versuchspflanzen visuell bonitiert, indem die Schädigung geschätzt wurde

Die erfindungsgemäßen Verbindungen (wie etwa die nach Beispiel 1, 2, 5 oder 11) erwiesen sich auch in diesem Versuch als gut wirksam.

17. <u>Prüfung auf wachstumsregulierende Wirkung Wuchshemmung bei Getreide</u>

In Schalenversuchen im Gewächshaus wurden junge Getreidepflanzen (Weizen, Gerste und Roggen) im 3-Blattstadium mit den zu prüfenden Verbindungen tropfnaß gespritzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Verbindungen (wie etwa nach Beispiel 1, 2, 5 oder 11) erwiesen sich als gut wirksam.

Patentansprüche

1. Imidazolinone der Formel I

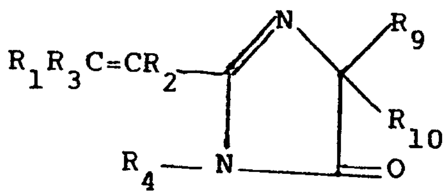

(Ia)

und

(I)

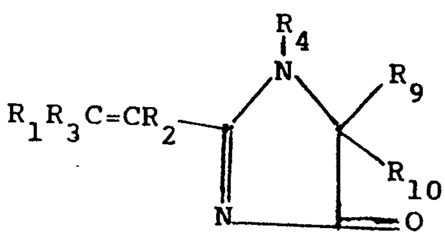

(Ib)

in denen

$R_1$ und $R_2$

unabhängig voneinander für gegebenenfalls substituiertes Niederalkyl,

$R_3$ für $COOR_5$, $COR_6$, CN, oder COOM

$R_4$ für H, gegebenenfalls substituiertes Niederalkyl, gegebenenfalls substituiertes Niederalkenyl oder $C_1$-$C_4$-Alkanoyl

$R_5$ für H, gegebenenfalls substituiertes $C_1$-$C_8$-Alkyl oder gegebenenfalls substituiertes Niederalkenyl, Phenyl oder $C_3$-$C_7$-Cycloalkyl,

$R_6$ für $NR_7R_8$, gegebenenfalls substituiertes Morpholinyl, gegebenenfalls substituiertes Pyrrolidinyl, gegebenenfalls substuiertes Piperidinyl, NH-$NH_2$, NH-N=$CR_{11}R_{12}$ oder

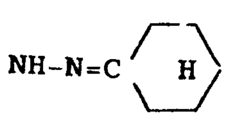

| | |
|---|---|
| $R_7$ und $R_8$ | unabhängig voneinander für H, gegebenenfalls substituiertes Niederalkyl, Niederalkenyl oder gegebenenfalls substituiertes Phenyl, $R_7$ auch für OH, |
| $R_9$ und $R_{10}$ | unabhängig voneinander für Niederalkyl |
| $R_{11}$ für | H oder Niederalkyl, |
| $R_{12}$ für | Niederalkyl, |
| M für | ein Äquivalent eines Alkali-, Ammonium- oder Erdalkaliions, oder |
| $R_3$ und $R_4$ | auch gemeinsam für -CO- stehen. |

2. Imidazolinone der Formel I'

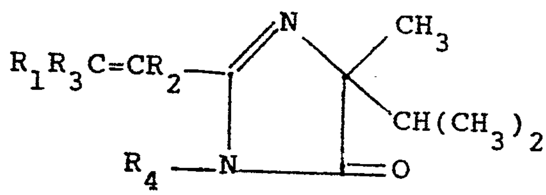

(Ia')

und Imidazolinone der Formel

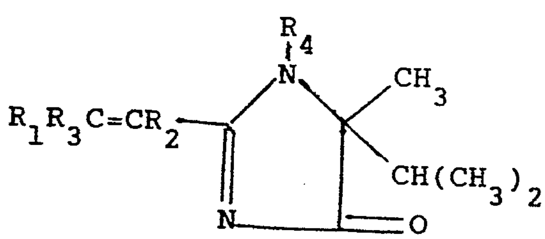

(Ib')

in denen

| | |
|---|---|
| $R_1$ und $R_2$ | unabhängig voneinander für Niederalkyl, |
| $R_3$ für | $COOR_5$, $COR_6$, CN, |
| $R_4$ für | H oder $C_1$-$C_4$-Alkanoyl, |
| $R_5$ für | H, Niederalkyl oder Niederalkenyl oder $C_5$-$C_7$-Cycloalkyl, |

$R_6$ für NR$_7$R$_8$, Morpholinyl, Pyrrolidinyl oder Piperidinyl,

$R_7$ und $R_8$ unabhängig voneinander für H, Niederalkyl, oder Phenyl, $R_7$ auch OH,

$R_3$ und $R_4$ gemeinsam auch für -CO- stehen.

3. Verbindungen der Formel I, worin $R_1$, $R_2$ und $R_9$ Methyl, $R_3$ $COOC_2H_5$, $COO\text{-}n\text{-}C_3H_7$, $COO\text{-}i\text{-}C_3H_7$, CO-N 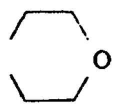 O oder $CON(CH_3)C_2H_5$, $R_4$ Wasserstoff und $R_{10}$ Ethyl oder Isopropyl bedeutet.

4. Verbindungen der Formel Ia", worin $R_1$, $R_2$ und $R_9$ Methyl bedeuten und $R_{10}$ für Ethyl oder Isopropyl steht.

5. Herbizide Mittel, gekennzeichnet durch einen Gehalt an einer Verbindung nach einem der Ansprüche 1 bis 4.

6. Verwendung von Verbindungen nach Anspruch 1 bis 4 bei der Bekämpfung unerwünschten Pflanzenwachstums.

7. Verfahren zur Bekämpfung von Unkräutern, dadurch gekennzeichnet, daß man Verbindungen nach Anspruch 1 bis 4 auf die Unkräuter und/oder ihren Lebensraum einwirken läßt.

8. Wachstumsregulatorisches Mittel, gekennzeichnet durch einen Gehalt an einer Verbindung nach Anspruch 1 bis 4.

9. Verwendung von Verbindungen nach Anspruch 1 bis 4 zur Beeinflussung des Pflanzenwachstums.

10. Verfahren zur Herstellung von Verbindungen nach Anspruch 1, 2, 3 oder 4, dadurch gekennzeichnet, daß man

a) eine Verbindung der Formel

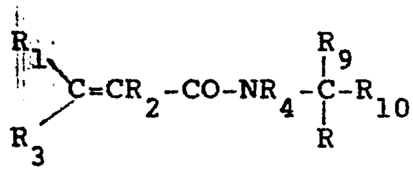

(II),

in der $R_1$, $R_2$, $R_3$, $R_4$, $R_9$ und $R_{10}$ die obige Bedeutung haben und R für $CONHR_4$ oder CN steht, mit der Maßgabe, daß die beiden Reste $R_4$ unterschiedliche Bedeutung haben können, cyclisiert
oder daß man

b) Verbindungen der Formel

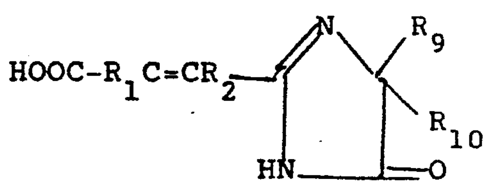

(III),

worin $R_1$, $R_2$, $R_9$ und $R_{10}$ die obige Bedeutung haben, zu Verbindungen der Formel Ia" cyclisiert
oder daß man

c) Verbindungen der Formel

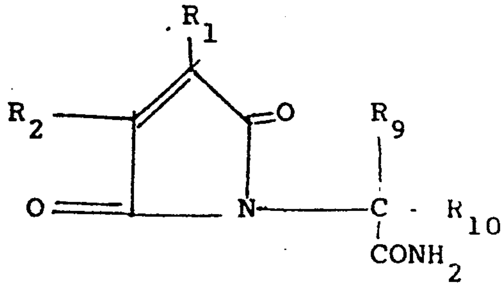

(IV)

worin $R_1$, $R_2$, $R_9$ und $R_{10}$ die obige Bedeutung haben, zu Verbindungen der Formel Ib" cyclisiert oder daß man

d) durch Ringöffnung bei Verbindungen der Formel Ia" bzw. Ib" mit Wasser bzw. wäßrigen Basen Verbindungen herstellt, in denen $R_3$ für COOH oder COOM steht, wobei M die obige Bedeutung hat,
oder daß man

e) durch Ringöffnung bei Verbindungen der Formel Ia" bzw. Ib" mit Alkoholen $R_5OH$ oder Aminen $R_6H$, in denen $R_5$ und $R_6$ die obige Bedeutung haben, entsprechende Verbindungen mit $R_3$ gleich $COOR_5$ bzw. $COR_6$ herstellt
oder daß man

f) zur Herstellung von Verbindungen der Formel I, in denen $R_4$ die obige Bedeutung hat, ausgenommen Wasserstoff, eine Verbindung der Formel

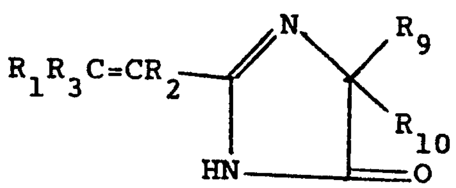

(Va)

bzw.

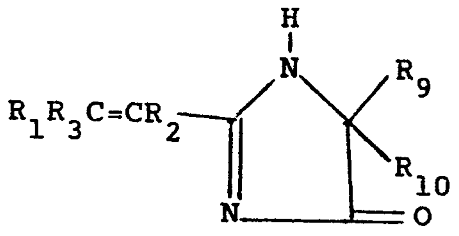

(Vb)

mit einer zur Einführung des Restes $R_4$ in der vorstehenden Bedeutung geeigneten Verbindung

$R_4X$ (VI)

g) eine Verbindung der Formel I, in der $R_3$ COOH bedeutet, nach üblichen Methoden in die Ester mit der Gruppe $COOR_5'$, worin $R_5'$ die gleichen Bedeutungen wie $R_5$ hat, ausgenommen Wasserstoff, oder in die Amide mit der Gruppe $COR_6$, worin $R_6$ die obige Bedeutung hat, überführt
oder daß man

h) Verbindungen der Formel I mit $R_3$ gleich COOH mit entsprechenden Basen MOH, worin M die obige Bedeutung hat, in die Salze mit der Gruppe COOM überführt.

**0226947**

Europäisches Patentamt

# EUROPÄISCHER RECHERCHENBERICHT

Nummer der Anmeldung

EP 86117183.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US - A - 3 894 008 (IRVINE)<br>* Anspruch 1 *<br>---- | 1 | C 07 D 233/70<br>C 07 D 487/04<br>A 01 N 43/50<br>A 01 N 43/90 |

| RECHERCHIERTE SACHGEBIETE (Int Cl 4) |
|---|
| C 07 D 233/00<br>C 07 D 487/00 |

Der vorliegende Recherchenbericht wurde für alle Patentansprüche erstellt

| Recherchenort | Abschlußdatum der Recherche | Prüfer |
|---|---|---|
| WIEN | 26-03-1987 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82